# EUROPEAN PATENT SPECIFICATION

(11) **EP 2 724 775 B1**
(45) Date of publication and mention of the grant of the patent: **19.02.2020**
(21) Application number: 12802201.9
(22) Date of filing: 21.06.2012
(51) Int. Cl.: B01J 13/02, A61K 9/64, B01J 13/04, A61K 38/00, A23P 10/30, A23L 33/105, A23L 33/135

(54) **METHOD FOR THE PRODUCTION OF MICRO-, SUB-MICRO- AND NANO-CAPSULES, BASED ON WHEY PROTEINS**
VERFAHREN ZUR HERSTELLUNG VON MIKRO-, SUBMIKRO- UND NANOKAPSELN AUF BASIS VON MOLKE PROTEINEN
PROCÉDÉ D'OBTENTION DE MICRO-, SUBMICRO- ET NANOCAPSUILES À PARTIR DE PROTÉINES DE PETIT LAIT

(30) Priority: 22.06.2011 ES 201131048 P
(43) Date of publication of application: 30.04.2014
(73) Proprietor: Consejo Superior De Investigaciones Científicas (CSIC), 28006 Madrid (ES)
(72) Inventor: LÓPEZ RUBIO, Amparo, E-46980 Paterna (Valencia) (ES); LAGARÓN CABELLO, José María, E-46980 Paterna (Valencia) (ES)
(74) Representative: Ungria López, Javier
(86) International application number: PCT/ES2012/070455
(87) International publication number: WO 2012/175776

(56) References cited:
- EP-A1- 0 093 668
- WO-A1-2004/110412
- WO-A1-2009/050333
- WO-A2-00/37547
- WO-A2-2008/146169
- US-A- 5 601 760
- US-A1- 2010 015 447
- ATMANE MADENE ET AL: "Flavour encapsulation and controlled release - a review", INTERNATIONAL JOURNAL OF FOOD SCIENCE AND TECHNOLOGY, BLACKWELL SCIENTIFIC PUBLICATIONS, OXFORD, GB, vol. 41, 1 January 2006 (2006-01-01), pages 1-21, XP002499388, ISSN: 0950-5423, DOI: 10.1111/J.1365-2621.2005.00980X
- JAWOREK ET AL: "Electrospraying route to nanotechnology: An overview", JOURNAL OF ELECTROSTATICS, ELSEVIER SCIENCE PUBLISHERS B.V. AMSTERDAM, NL, vol. 66, no. 3-4, 28 January 2008 (2008-01-28), pages 197-219, XP022510125, ISSN: 0304-3886, DOI: 10.1016/J.ELSTAT.2007.10.001
- YEO Y. ET AL.: 'Microencapsulation Methods for Delivery of Protein Drugs.' BIOTECHNOLOGY BIOPROCESS ENGINEERING vol. 6, no. 4, 2001, pages 213 - 230, XP008153068
- FUKUI Y. ET AL.: 'Preparation of monodispersed polyelectrolyte microcapsules with high encapsulation efficiency by and electrospay technique.' COLLOIDS AND SURFACES A: PHYSICOCHEMICAL AND ENGINEERING ASPECTS. vol. 370, no. 1-3, 2010, pages 28 - 34, XP027404116

## Description

The present invention relates to a method for the production of micro-, sub-micro- and nano-capsules based on whey proteins. These micro-, sub-micro- and nano-capsules produced may be used as encapsulation vehicles for ingredients with added value and functional additives, to be used in multi-sector applications, including food and pharmaceutical preparations.

### STATE OF THE ART

There is considerable interest in the development of biopolymeric particles from proteins and polysaccharides, as they may be used to protect and release, in a controlled manner, bioactive compounds in food and pharmaceutical systems (Jones, O., Decker, E.A., McClements, D.J. (2010). Food Hydrocolloids 24, 239-248). A large variety of methods for protein microparticle production have been developed. The most common techniques are spray-drying *(*Bruschi, M.L., Cardoso, M.L.C., Lucchesi, M.B. & Gremiao, M.P.D. (2003). Gelatin microparticles containing propolis obtained by spray-drying technique: Preparation and characterization. International Journal of Pharmaceutics, 264, 45-55*),* emulsification and crosslinking (Ishizaka, T., & Koishi, M. (1981). Preparation of egg albumin microcapsules and microspheres. Journal of Pharmaceutical Sciences, 70, 358-361*)* or coacervation *(*Mauguet, M.C., Legrand, J., Brujes, L., Carnelle, G., Larre, C., & Popineau, Y.J. (2002). Gliadin matrices for microencapsulation processes by simple coacervation method. Journal of Microencapsulation, 19, 377-384*)*. Nonetheless, these techniques require the solutions to be heated or organic agents to be used in at least one of the phases of production, thus leading to the destruction of encapsulated sensitive ingredients, as well as to toxicity problems associated with residual content of the organic agents (Birnbaum, D., Kosmala, J., Henthorn, D., & Brannon-Peppas, L. (2000). Controlled release of beta-estradiol from PLAGA microparticles: The effect of organic phase solvent on encapsulation and release. Journal of Controlled Release, 65, 375-387*)*. Therefore, new technologies that do not involve harsh conditions (both in terms of temperature and in terms of the solvents used), which furthermore result in smaller particle sizes, are highly desirable. In this way, electro-spinning and electro-spraying techniques (electro-spinning and electro-spraying at high-voltage) and blow-spinning and blow-spraying techniques (spinning and spraying by means of blowing) are simple and highly versatile methods for producing encapsulates with controlled morphology, such as micron and submicron range fibres and/or capsules by means of the action of an external electrical field applied between two electrodes or a pressurized fluid flow, to which the polymeric solution is subjected. The blow spinning/spraying technique does not even require the use of electrical fields. It is therefore based on applying a pressurized gas at high speed in order to produce the micro- and nano-structures. These processes do not require the use of temperature. Furthermore, the biopolymers (such as proteins) may be formed from aqueous solutions, simply by means of adjusting the parameters of the process correctly and/or by means of varying the properties of the solution by adding suitable additives. The electro-spinning technique has been widely used in order to generate nano-fibres, being applied in various fields such as regenerative medicine, catalysis or filtration *(*Subbiah, T., Bhat, G.S., Tock, R.W., Parameswaran, S., and Ramkumar, S.S. (2005). Electrospinning of nanofibers. Journal of Applied Polymer Science, 96, 557-569*)* but it also has high potential in the food technology sector in order to develop new functional foods, as has been recently demonstrated *(*Torres-Giner, S., Martinez-Abad, A., Ocio, M.J., and Lagaron, J.M. (2010). Stabilization of a nutraceutical omega-3 fatty acid by encapsulation in ultrathin electrosprayed zein prolamine. Journal of Food Science, 75, N69-N79*;* Lopez-Rubio, A., Sanchez, E., Sanz, Y., and Lagaron, J.M. (2009). Encapsulation of living bifidobacteria in ultrathin PVOH electrspun fibers. Biomacromolecules, 10, 2823-2829*)*. Nonetheless, the blow spinning and blow spraying techniques have been recently developed and their application in biopolymers has been fairly limited up to now *(*Medeiros, E.S., Glenn, G.M., Klamczynski, A.P., Orts, W.J., Maltoso, L.H.C. (2009). Solution blow spinning: a new method to produce micro- and nanofibers from polymer Solutions. Journal of Applied Polymer Science 113, 2322-2330*;* Sinha-Ray, S., Zhang, Y., Yarin, A.L., Davis, S.C., Pourdeyhimi, B. (2011). Solution blowing of soy protein fibers. Biomacromolecules 12, 2357-2363*)*.

The morphology of the structures produced by means of these spinning techniques may be modified by setting the parameters of the process, and for a certain material, very small capsules may be obtained (process known as "spraying" owing to the discontinuous nature of the structures obtained).

Nowadays, there is no bibliographic reference describing the development of micro- or nano-capsules from milk proteins using the techniques described (electro-spinning/electro-spraying and blow spinning/blow spraying). The use of milk proteins to encapsulate has barely been explored, especially those based on the concentrate. However it has great potential owing to the excellent functional properties of said proteins and their low cost (given that they are a by-product generated during the production of fermented milk products). The background scientific literature contains several examples of microencapsulates from these milk protein concentrates, using techniques such as spray-drying *(*Rosenberg, M., Young, S.L., Brooker, B.E., and Colombo, V.E. (1993). Food Structure 12, 31-41*;* Bylaite, E., Venskutonis, P.R., Mapdpieriene, R. (2001). European Food Research and Technology 212, 661-670*;* Jimenez, M., Garcia, H.S., Beristain, C.I. (2010). Journal of Food Process Engineering 33, 434-447; Huynh, T. V., Caffin, N., Dykes, G.A., Bhandari, B. (2008). Drying Technology 26, 357-368*;* Jafari, S.M., Assadpoor, E., Bhandari, B., He, Y. (2008). Food Research International 41, 172-183*;* Hogan, S.A., McNamee, B.F., O'Riordan, E.D., O'Sullivan, M. (2001). Journal of Food Science 66, 675-680*.) and hydrogel use (*Gunasekaran, S., Ko, S., and Xiao, L. (2007). Journal of Food Engineering 83, 31-40*).* There is also a patent describing the obtaining of micro-particles from milk protein concentrate but involving the use of temperature and high pressures (WO/2008/06). Another patent describes the production of liposomes microencapsulates and a whey protein concentrate produced by homogenization, pasteurization and spray drying process (WO/2009/050333(A1)*).*

More specifically, WO2009/050333 A1 states that orally administered liposomes were not stable enough, since low pH of the stomach and the presence of bile salts and lipases tend to destabilize the active substance-liposome complex (see page 3, lines 18-21). In order to increase the stability of such liposomes, this document provides a microencapsulated liposomal composition comprising biologically active lipids and a modified whey protein mixture, wherein such mixture comprises 60 - 95 wt% of whey protein and 5 - 40 wt% of whey protein modified by sulfitolysis (see page 6, lines 21-26, page 10, lines 10-16, page 11, lines 16-20 and claim 1). According to this prior art document, the microencapsulated liposomal composition therein described is obtained by a method comprising several heat treatments. First of all, a mixture of whey protein and modified whey protein is dissolved in water by stirring at a temperature of 30-75°C, preferably 40-70°C. Once biologically active lipids are added to the mixture, a lipid-water suspension is obtained heating to a temperature of 30-80°C, preferably 45-70°C. After that, an emulsion is obtained and, optionally, pasteurized at a temperature of 65-85°C, preferably 70-80°C. Finally, the emulsion is spray dried or lyophilizated, preferably in a spray dryer at an inlet temperature of 150-180°C and outlet temperature of 70-90°C, using air or under an inert atmosphere (see page 12, line 22 to page 13, line 20).

Furthermore, Atmane et al. ("Flavour encapsulation and controlled release - a review", International Journal of Food Science and Technology, Blackwell Scientific Publications, Oxford, GB (2006), 41(1), pages 1-21) describes different materials and processes to encapsulate flavours. In particular, this document mentions that whey protein isolates can be used for microencapsulation of volatiles by spray drying (see page 5, right column, second paragraph). This document also describes that a combination of whey protein and carbohydrates can be used as carrier material in the encapsulation of volatile components. In such system, whey protein acts as emulsifying and film-forming agent, whereas carbohydrates act as the matrix-forming material (see page 6, left column, second). Besides that, US5601760 A describes a method for microencapsulation of various core materials using whey proteins microencapsulating agents, and spray drying as microencapsulating technique. In particular, this prior art document discloses that drying conditions usually consists of an inlet air temperature of about 100°C and about 210°C, and of an outlet air temperature from about 50°C to about 140°C (see column 10, lines 50-56).

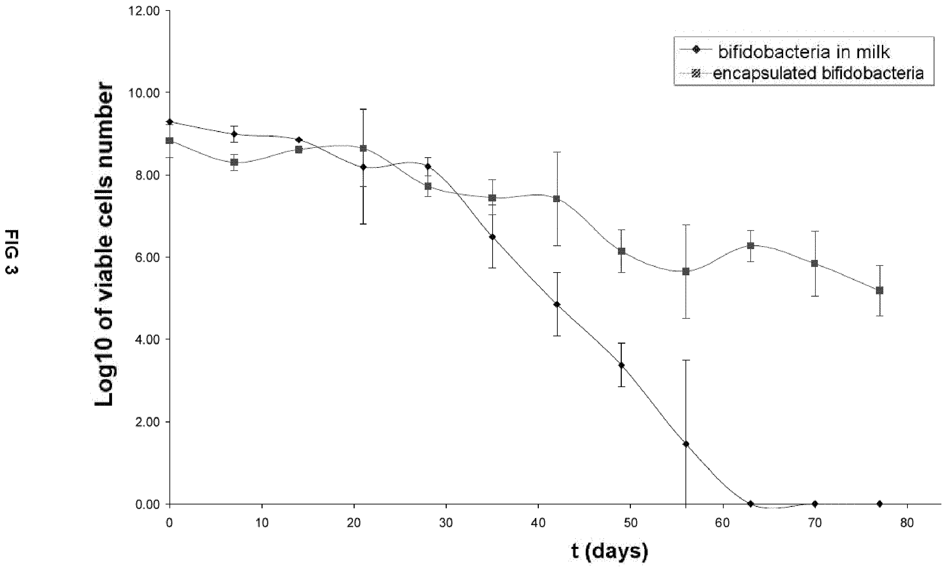

On the other hand, Jaworek et al. ("Electrospraying route to nanotechnology: An overview", Journal of Electrostatics, Elservier Science Publishers B.V. Amsterdam, NL, (2008), Vol. 66, no. 3-4, pages 197-219) provides some examples of electrospraying or electrospinning of proteins to produce microcapsules or nanocapsules (see item 3.5; and tables 5, 6). In particular, this document refers to reference [227] (see table 5), wherein Bovine serum albumin (BSA) is used as the encapsulated material at the core, whereas poly-L-lactice (PLA) is used as the encapsulating material at the shell. Bovine serum albumin (BSA) is also used in reference [232] (see table 5), wherein the production of thick capsules (10-20 microns) from an ethanol/water mixture is described. Besides that, Jaworet et al. also cites reference [255] (see table 6), wherein aqueous solutions of alfa-lactoalbumin protein were processed by the technique of electrospray deposition (ESD), a technique that, in a dry chamber, makes use of a glass not conductive capillary injector which has a platinum electrode, to produce under certain conditions a protein film that is later crosslinked to form a continuous materials with certain porosity to create thin films with biological activity (see, in particular, figure 3 of reference [255] (I. Uematsu et al., "Surface morphology and biological activity of protein thin films productd by electrospray deposition", Journal of Colloid and Interface Science 269 (2004), pages 336-340), wherein films therein obtained are reproduced.

In addition to the above-mentioned, WO00/37547 A2 refers to a production method to create expandable hollow polymeric microspheres to obtain foamed materials, and US2010/015447 A1 makes reference to the electrospraying technology to develop Janus structures (i.e., two solid phase materials), which combined side by side provide stability to the process.

### DESCRIPTION OF THE INVENTION

The present invention proposes the use of products based on whey proteins and the mixture thereof with other biopolymers, in order to develop micro- (over one micron) submicro- (under one micron, up to 100 nanometres) and nano-capsules (between 1 and 100 nanometres), which serve to protect substances with added value.

Furthermore, in order to resolve the technical problems derived from micro-, submicro- and nano-capsule production, hereinafter referred to as capsules, by means of conventional methods for producing the same, the present invention describes a method for obtaining said capsules, without the technical inconvenience of using high temperatures and pressures, organic solvents or other relatively harsh conditions, which might affect stability and by means of which ingredients sensitive to environmental conditions may be encapsulated, for which encapsulation is a valuable protection process.

Therefore, a first aspect of the present invention refers to a method for obtaining capsules from whey proteins, comprising the following steps:
a) diluting the product based on whey proteins in water;
b) adding the following to the solution of step a):
   i) functional ingredients to be encapsulated that can be dissolved or suspended in water; or
   ii) a solution of the ingredient to be encapsulated; and
c) electro-spinning or electro-spraying or blow spinning or blow spraying the solution resulting from step b), wherein
   ∘ the electrospinning or electrospraying is carried out with a distance between the capillary and the support of between 2 and 50 cm, a deposition rate range per needle from 0.01 to 10 ml/h and a voltage of between 0.1 and 1000 kV, and
   ∘ the blow spinning or blow spraying is carried out with a distance between the capillary and the support of between 2 and 50 cm, a deposition rate range per needle from 0.01 to 10 ml/h and by applying a flow of pressurized gas at between 200 and 300 m/s.

In a preferred, non-limiting embodiment, the protein preparation is based on a whey protein concentrate from mammal or soy. Preferably, from cow's, sheep, goat or soy milk, and even more preferably from cow.

In a preferred embodiment, the percentage by weight of the proteins in the solution varies from 0.1 to 99 %, preferably between 10 and 70 %.

According to another preferred embodiment, the ingredients are selected from the group formed by antioxidants (vitamin C, vitamin E, carotenoids, phenolic compounds such as flavonoids and resveratrol) and natural or synthetic antioxidant concentrates or isolates, biological organisms such as cells of biomedical value and probiotics (lactic bacteria, bifidobacteria), prebiotics (lactulose, galacto-oligosaccharides, fructo-oligosaccharides, maltooligosaccharides, xylo-oligosaccharides and oligosaccharides from soy), symbiotics, fibres, oleic acid, polyunsaturated fatty acids (omega-3 and omega-6) and other marine oils, phytosterols, phytoestrogens, ingredients of a protein based nature (DNA and its derivatives, lactoferrin, ovotransferrin, lactoperoxidase, lysozyme, soy protein, immunoglobulin, bioactive peptides) and pharmaceutical products such as nutraceuticals and other preparations and substances of added value for the pharmaceutical, biomedical, food and chemical industries, which may be destabilized by environmental conditions, processing or storage in their commercial presentation, or any combination thereof.

The ingredients are more preferably selected from the group formed by:
- carotenoids and polyphenols,
- bifidobacteria and lactic bacteria,
- cells of biomedical interest for bone and tissue regeneration,
- polyunsaturated fatty acids,
- enzymes and other proteins of technological value selected from lactoferrin, ovotransferrin, lactoperoxidase, lysozyme, soy protein and immunoglobulin,
- bioactive peptides selected from anti-hypertensives and antimicrobials. More preferably still:
- bifidobacteria and lactic bacteria,
- cells of biomedical interest for bone and tissue regeneration,
- polyunsaturated fatty acids,
- enzymes and other proteins of technological value selected from lactoferrin, ovotransferrin, lactoperoxidase, lysozyme, soy protein and immunoglobulin.
- bioactive peptides, selected from anti-hypertensives and antimicrobials.

In another preferred embodiment, other biopolymers selected from the following list: carbohydrates, other proteins and lipids, are added in step a) in order to produce mixtures that improve stability and protection of the encapsulated functional ingredients.

In another preferred embodiment, additives selected from the following list: plasticizers, crosslinking agents, surfactants, acids, alkalis, emulsifiers, antioxidants, processing aids in general or any mixture thereof or others which facilitate capsule formation or the incorporation of the ingredients to be encapsulated, are added to step a.

In another preferred embodiment, a homogenizing step by means of stirring and/or ultrasound is performed after step b). Stirring may be vigorous so as to facilitate the dispersion of the ingredient to be encapsulated and/or of the additives in the biopolymeric matrix (whey protein concentrate).

According to another preferred embodiment, in step c) the electro-spinning or electro-spraying is performed by applying a voltage preferably of between 5 and 30 kV.

A second aspect of the present invention refers to the capsules produced by means of the method described above.

A third aspect of the present invention refers to a functional food comprising the capsules with the functional ingredients, obtained by means of the method described above.

A fourth aspect of the present invention refers to a pharmaceutical or biomedical composition comprising the capsules with the ingredients, obtained by means of the method described above.

A fifth aspect of the present invention refers to a chemical composition (e.g. fertilizers, phytosanitaries, antimicrobials, anti-odour, fragrances, active agents and for products suspended or dispersed in non-polar solvents), comprising the capsules with the ingredients obtained by means of the method described above.

A sixth aspect of the present invention refers to a nutraceutical composition comprising the capsules with the ingredients obtained by means of the method described above.

A seventh aspect of the present invention refers to a functional container comprising the capsules with the ingredients obtained by means of the procedure described above.

An eighth aspect of the present invention refers to the use of the capsules obtained by means of the method described above, for the incorporation thereof in pharmaceutical, biomedical, chemical, nutraceutical or food compositions or in functional containers.

In the present invention, the electro-spinning/electro-spraying techniques refer to a technology based on applying high electrical fields to produce electrically charged fluids from viscoelastic polymeric solutions, which, upon drying, produce micro-fibres, nano-fibres and nano-capsules, respectively.

On the other hand, blow spinning and blow spraying use a fluid (usually a gas at a high speed and pressure) for generating fibres and capsules of sub-micrometric size.

There techniques do not require the use of high temperatures nor of organic solvents and encapsulation structures may be generated from aqueous solutions of proteins (in this case, specifically from a whey protein concentrate) and mixtures thereof.

In the present invention, the term "functional container" refers to that container material containing bioactive ingredients (in this specific case, it would contain the capsules or fibres developed, incorporated into its structure or as an internal coating of the container) preserved in optimal conditions until it is released to packaged foods, thus contributing to the production of functional foods.

In the present invention, the term "bioactive ingredient" refers to a compound or ingredient which has a beneficial effect on health or high added-value in a technical field. Likewise, it may refer to extracts or chemical compounds denominated functional, even to those obtained from common foods. Examples of ingredients to which functional properties are attributed are olive oil, red wine, broccoli, soy, bifidobacteria, β-carotenes, etc.

In the present invention, the term "functional food" refers to those foods that are not only prepared for their nutraceutical properties, but also to fulfil a *specific function,* as it might be to improve health or reduce the risk of developing diseases. For this reason, biologically active components, such as minerals, vitamins, fatty acids, food fibre or antioxidants, etc., are added to them.

In the present invention, the term "nutraceutical" refers to a concentrate of functional or bioactive ingredients, which serve to fulfil a *specific function,* as it might be to improve health and reduce the risk of developing diseases.

The pharmaceutical or biomedical composition is a set of components formed by at least the micro- or nano-capsules of the invention, which have at least one application for improving the physical, physiological or psychological well-being of an individual, implying an improvement in their general state of health, for example a cosmetic application, although it may not imply a physiological effect on the organism and rather implies an improvement in the individual's well-being related to their psychology. Therefore, said pharmaceutical composition may be a personal hygiene product, a cosmetic product or a product that may form the base for producing said products or the base for producing a medicine, an implant or a biomedical device.

The tern "personal hygiene product" refers to the substances or preparations which, not being legally considered to be drugs, sanitary products, cosmetic products or biocides, are intended to be applied to the skin, teeth or mucous membranes of the human body, with an hygienic or aesthetic purpose or to neutralize or eliminate ectoparasites.

The term "cosmetic product" refers to all substances or preparations intended to be put into contact with the diverse superficial parts of the human body (epidermis, hair or capillary system, nails, lips and external genital organs) or with the teeth or oral mucosa, with the exclusive or main purpose of cleaning them, perfuming them, modifying their aspect and/or correcting body odours and/or protecting or keeping them in good condition.

The term "drug" has a more limited meaning than the term "pharmaceutical composition", as defined in the present invention, given that the term drug necessarily implies a therapeutic effect, i.e. a physiological effect on the metabolism of the subject.

Throughout the description and claims the term "comprises" and its variants are not intended to exclude other technical features, additives, components or steps. For those skilled in the art, other objects, advantages and characteristics of the invention will be deduced, partly from the description and partly from the practice of the invention. The examples and figures below provide a non-limiting illustration of the present invention.

### FIGURES

**Figure 1** shows an image of a Scanning Electron Microscopy (SEM) of electro-sprayed structures from a whey protein concentrate in an aqueous solution.
**Figure 2** shows an image of an optical microscopy obtained with polarized light (A), using a florescent source (B) for electro-sprayed structures of a whey protein concentrate, containing the antioxidant β-carotene dissolved in glycerol.
**Figure 3** shows a semi-logarithmic graph of the number of units that form a colony per millilitre of bifidobacteria, both encapsulated and un-encapsulated (in milk), at various time intervals, stored at 20°C.
**Figure 4** shows an image of an optical microscopy obtained using polarized light for structures obtained by means of blow spraying of a whey protein concentrate.

### EXAMPLES

Below, the invention is illustrated by means of a number of trials carried out by the inventors, demonstrating the efficacy of the invention method in obtaining nano-capsules from a whey protein concentrate, and the ability of these matrices to protect various ingredients.

### Example 1.

### Obtaining nano-capsules from a whey protein concentrate

In this example, a typical process for obtaining nano-capsules based on a whey protein concentrate is described, using the electro-spraying technique.

In a first step, the solution of the whey protein concentrate is prepared in distilled water. The concentration of the protein concentrate used is 40% by weight relative to the volume of solvent. It is stirred at room temperature until a homogenous solution is obtained.

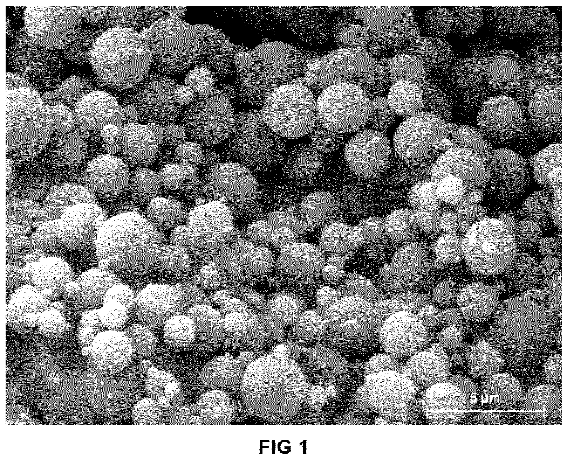

Once the solution has been obtained, the same is used to generate the micro- and nano-capsules by means of the electro-spraying technique with a horizontal configuration. The solution is introduced in 5 ml syringes, connected to a stainless steel needle of 0.9 mm in diameter, via Teflon tubes. The needle is connected to an electrode, which is in turn connected to a 0-30 kV power source. A voltage comprised between 12-14 kV is applied and the solution is pumped through said needle with a flow of 0.3 ml/h. The counter-electrode is connected to a stainless steel plate (collector), where the structures obtained are collected, the distance between the needle and the collector being 7 cm. The process is carried out at room temperature. The micro- and nano-capsules shown in Figure 1 are obtained in this way.

The size of the capsules obtained in this way therefore varies between 50 nm and 3 microns, although nano-capsules (i.e. capsules smaller than 100 nm) are preferably obtained.

### Example 2.

### Protecting the antioxidant β-carotene by means of encapsulation, using matrices made from whey protein concentrate

In this example, the encapsulation ability of the structures generated by means of electro-spraying and the ability thereof to protect sensitive ingredients such as the antioxidant β-carotene are demonstrated.

Firstly, a solution of the antioxidant β-carotene was prepared in glycerol, proven to be highly capable of stabilizing this bioactive ingredient against photodegradation. 1 g of β-carotene was dissolved in 10 ml of glycerol and it was left being stirred at room temperature for 24 hours. Separately, a solution of whey protein concentrate was prepared in distilled water, using a concentration of 40% by weight relative to the volume of the solvent and it was stirred at room temperature until a homogenous solution was obtained. The β-carotene dissolved in glycerol was added to the latter solution, the proportion added constituting 20% by weight relative to the weight of the protein used. It was left being stirred for a couple of hours at room temperature in order to achieve a homogenous solution.

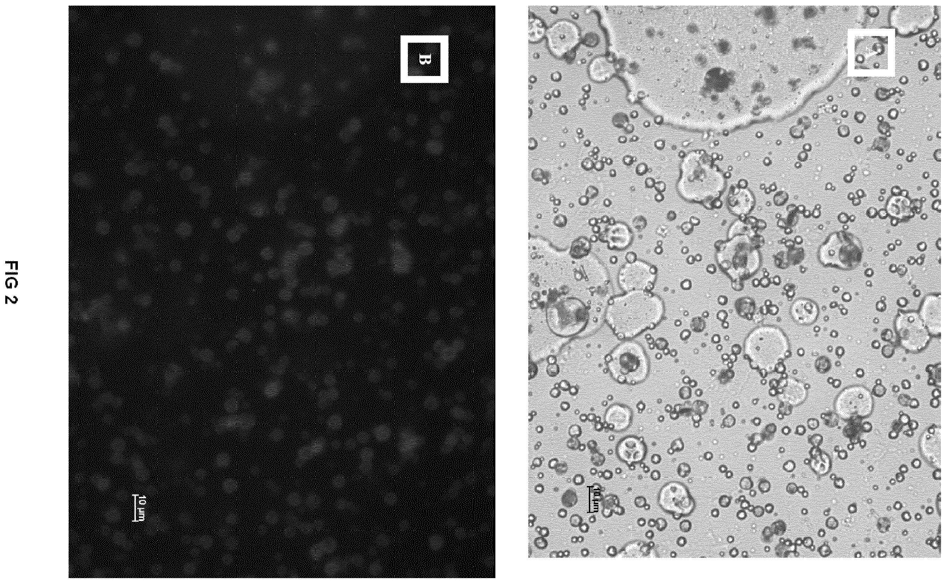

This latter aqueous solution containing the whey protein concentrate and the antioxidant β-carotene dissolved in glycerol was used to produce the micro-capsules shown in Figure 2 by means of the electro-spraying technique with a horizontal configuration. The solution was introduced into 5 ml syringes, connected to a stainless steel needle of 0.9 mm in diameter, via Teflon tubes. The needle was connected to an electrode, which in turn was connected to a 0-30 KV power source. A voltage comprised between 9-10 kV was applied and the solution was pumped through said needle with a flow of 0.3 ml/h. The counter-electrode was connected to a stainless steel plate (connector), where the structures obtained were collected, the distance between the needle and the collector being 7 cm. The process was carried out at room temperature. Some of the capsules obtained were collected directly on a glass support for microscopy. The average size of the capsules in this case was greater (∼ 4 microns) owing to the presence of glycerol in the solution.

### Example 3.

### Encapsulation and stabilization of bifidobacteria at 20°C by means of the use of electro-spraved microcapsules, made from a whey protein concentrate

In this example, the ability of this technology to develop structures of whey protein concentrate to encapsulate and protect bifidobacteria of interest in the development of new functional food products is demonstrated.

The viability study was carried out using a commercial bifidobacteria strain and a concentrated solution of bacteria in skimmed milk was used as control, known to act as a natural protection agent for bifidobacteria.

The solution of the whey protein concentrate was firstly prepared as described in the above examples, i.e. mixing a 40% proportion by weight of the protein concentrate in the solvent by means of stirring at room temperature. The difference in this example lies in that rather than using distilled water as a solvent, milk containing a known concentration of bifidobacteria cells was used directly. This solution was kept stirred at room temperature until a homogenous mixture was obtained, which was used to produce micro-capsules by means of electro-spraying. The solution was introduced into 5 ml syringes, connected to a stainless steel needle of 0.9 mm in diameter, via Teflon tubes. The needle was connected to an electrode, which was in turn connected to a 0-30 kV power source. A voltage comprised between 12-14 kV was applied and the solution was pumped through said needle with a flow of 0.6 ml/h. The counter-electrode was connected to a stainless steel plate (collector), where the structures obtained were collected, the distance between the needle and the collector being 6 cm. The process was carried out at room temperature. The material collected in the collector was divided in eppendorfs, stored at 20°C, to the viability count over time. They were compared to the bifidobacteria cells suspended in skimmed milk and stored in the same conditions.

The encapsulation technique by means of electro-spraying of the bifidobacteria, using a whey proteins concentrate as an encapsulating matrix, proved to increase viability of the encapsulated cells at room temperature, in comparison to the cells suspended in skimmed milk, as shown in Figure 3.

### Example 4.

### Obtaining sub-micrometric encapsulation structures from a whey protein concentrate, using the blow spinning/ blow spraying technique

In this example, the method for producing sub-micrometric capsules by means of the blow spinning/ blow spraying technique is detailed.

A solution of the milk protein concentrate is firstly prepared in water, using a concentration of the same at 40% by weight relative to the volume used, stirring at room temperature until a homogenous solution is obtained.

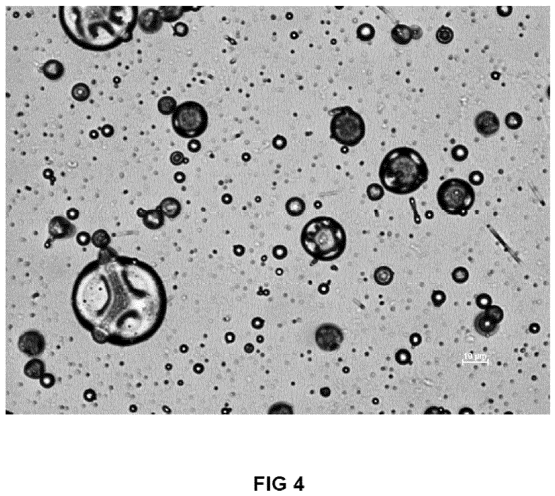

This aqueous solution containing the whey protein concentrate is used to generate the micro-capsules shown in Figure 2 by means of the electro-spraying technique by blowing with a vertical configuration. The solution was introduced into a 5 ml syringe located in a syringe pump and connected to an internal stainless steel needle of 0.9 mm in diameter, via Teflon tubes. This needle was mounted in coaxial configuration, the external needle being that through which pressurized nitrogen gas flowed at a high rate (230 -250 m/s). The nitrogen flow pumped coaxially through the external needle accelerates and spins the protein solution flowing through the internal needle and assists the formation of the encapsulation structures. The flow of the solution with the milk protein concentrate was 0.5 ml/h. The nitrogen gas pressure in the bottle was 20-30 bars. The structures produced and solidified were collected in a collector located 18-20 cm away. Figure 4 shows an image of an optical microscopy of the capsules generated.

## Claims

1. Method for obtaining capsules from a whey protein product, comprising the following steps:
a) diluting the whey protein product in water,
b) adding the following to the solution of step a):
i. functional ingredients to be encapsulated which are capable to be dissolved or suspended in water, or
ii. a solution of the ingredient to be encapsulated; **characterised in that** the method also comprises:
c) electrospinning or electrospraying or blow spinning or blow spraying the solution produced in step b), wherein
∘ the electrospinning or electrospraying is carried out with a distance between the capillary and the support of between 2 and 50 cm, a deposition rate range per needle from 0.01 to 10 ml/h and a voltage of between 0.1 and 1000 kV, and
∘ the blow spinning or blow spraying is carried out with a distance between the capillary and the support of between 2 and 50 cm, a deposition rate range per needle from 0.01 to 10 ml/h and by applying a flow of pressurized gas at between 200 and 300 m/s.

2. The method according to claim 1, wherein the product based on whey proteins is a concentrate of whey proteins from mammals.

3. The method according to claim 2, wherein the product based on whey proteins is a concentrate of whey proteins from cow's, sheep or goat's milk.

4. The method according to claim 3, wherein the product based on whey proteins proceed from a concentrate of whey proteins from cow's milk.

5. The method according to any of the claims 1 to 4, wherein the percentage by weight of the protein in the solution ranges from 0.1 to 99%.

6. The method according to any of the claims 1 to 5, wherein the percentage by weight of the proteins in the solution ranges from 10 to 70%.

7. The method according to any of the claims 1 to 6, wherein the ingredients are functional ingredients selected from the group of antioxidants, probiotics, cells for bone and tissue regeneration, prebiotics, symbiotics, fibres, oleic acid, polyunsaturated fatty acids, marine oils, phytosterols, phytoestrogens, functional ingredients of protein nature, nutraceuticals, enzymes or proteins of technological value selected from lactoferrin, ovotransferrin, lactoperoxidase, lysozyme, soy protein, immunoglobulin or any combination thereof.

8. The method according to any of the claims 1 to 7, wherein the functional ingredients are selected from the group of:
a) β-carotene,
b) bifidobacteria and lactic bacteria,
c) cells of biomedical interest for bone and tissue regeneration,
d) polyunsaturated fatty acids,
e) enzymes and other proteins of technological value selected from lactoferrin, ovotransferrin, lactoperoxidase, lysozyme, soy protein, immunoglobulin,
f) bioactive peptides selected from anti-hypertensive and anti-microbial peptides.

9. The method according to any of the claims 1 to 8, wherein other biopolymers selected from the following list: carbohydrates, proteins and lipids, are added in step a).

10. The method according to any of the claims 1 to 9, wherein additives selected from the following list: plasticizers, acids, crosslinking agents, alkalis, emulsifiers, antioxidants, agents that assist the process or any mixture thereof or other agents that facilitate the formation of the capsules or the incorporation of the ingredients to be encapsulated, are added in step a).

11. The method according to any of the claims 1 to 10, wherein, following step b), a homogenisation step is carried out by means of stirring and/or ultrasound.

12. The method according to any of the claims 1 to 11, wherein in step c) the electrospinning or electrospraying is carried out by applying a voltage of between 5 and 30 kV.

13. Capsules obtainable by the method of claims 1 to 12.

14. Use of the capsules of claim 13, to prepare chemical compositions or functional containers.

15. Use of the capsules of claim 14, wherein the chemical compositions are selected from the group consisting of functional food products, pharmaceutical compositions, biomedical compositions and nutraceutical compositions.

## Patentansprüche

1. Verfahren zur Gewinnung von Kapseln aus einem Molkeproteinprodukt, umfassend die folgenden Schritte:
a) Verdünnung des Molkeeiweißerzeugnisses in Wasser,
b) der Lösung aus Schritt a) Folgendes Hinzufügen:
i. funktionelle Bestandteile, die eingekapselt werden sollen und die in der Lage sind in Wasser gelöst oder suspendiert zu werden, oder
ii. eine Lösung des Bestandteils, der eingekapselt werden soll, dadurch charakterisiert, dass das Verfahren auch umfasst:
c) Elektrospinnen oder Elektrosprühen oder Blasspinnen oder Blassprühen der in Schritt b) hergestellten Lösung, wobei
∘ das Elektrospinnen oder Elektrosprühen mit einem Abstand zwischen der Kapillare und dem Träger zwischen 2 und 50 cm, ein Abscheidegeschwindigkeitsbereich pro Nadel von 0,01 bis 10 ml/h und einer Spannung zwischen 0,1 und 1000 kV durchgeführt wird und
∘ das Blasspinnen oder Blassprühen mit einem Abstand zwischen der Kapillare und dem Träger zwischen 2 und 50 cm, ein Abscheidegeschwindigkeitsbereich pro Nadel von 0,01 bis 10 ml/h und durch Anwenden eines Druckgasflusses zwischen 200 und 300 m/s durchgeführt wird.

2. Verfahren gemäß Anspruch 1, wobei das auf Molkeproteinen basierende Produkt ein Konzentrat von Molkeproteinen von Säugern ist.

3. Verfahren gemäß Anspruch 2, wobei das auf Molkeproteinen basierende Produkt ein Konzentrat von Molkeproteinen aus Kuh-, Schaf- oder Ziegenmilch ist.

4. Verfahren gemäß Anspruch 3, wobei das auf Molkeproteinen basierende Produkt aus einem Konzentrat von Molkeproteinen aus Kuhmilch hervorgeht.

5. Verfahren gemäß einem der Ansprüche 1 bis 4, wobei der Gewichtsprozentsatz des Proteins in der Lösung im Bereich von 0,1 bis 99 % liegt.

6. Verfahren gemäß einem der Ansprüche 1 bis 5, wobei der Gewichtsprozentsatz der Proteine in der Lösung im Bereich von 10 bis 70 % liegt.

7. Verfahren gemäß einem der Ansprüche 1 bis 6, wobei die Bestandteile funktionelle Bestandteile sind, die ausgewählt sind aus der Gruppe der Antioxidantien, Probiotika, Zellen für die Knochen- und Geweberegeneration, Präbiotika, Symbiotika, Fasern, Ölsäure, mehrfach ungesättigten Fettsäuren, Meeresöle, Phytosterine, Phytoöstrogene, funktionellen Inhaltsstoffe mit Proteincharakter, Nutrazeutika, Enzyme oder Proteine von technologischem Wert, ausgewählt aus Lactoferrin, Ovotransferrin, Lactoperoxidase, Lysozym, Sojaprotein, Immunglobulin oder einer Kombination davon.

8. Verfahren gemäß einem der Ansprüche 1 bis 7, wobei die funktionellen Bestandteile ausgewählt sind aus der Gruppe von:
a) β-Carotin,
b) Bifidobakterien und Milchsäurebakterien,
c) Zellen von biomedizinischem Interesse für die Knochen- und Geweberegeneration,
d) mehrfach ungesättigten Fettsäuren,
e) Enzymen und anderen Proteinen von technologischem Wert, ausgewählt aus Lactoferrin, Ovotransferrin, Lactoperoxidase, Lysozym, Sojaprotein, Immunglobulin,
f) bioaktiven Peptiden, ausgewählt aus blutdrucksenkenden und antimikrobiellen Peptiden.

9. Verfahren gemäß einem der Ansprüche 1 bis 8, wobei andere Biopolymere, ausgewählt aus der folgenden Liste: Kohlenhydrate, Proteine und Lipide, in Schritt a) zugegeben werden.

10. Verfahren gemäß einem der Ansprüche 1 bis 9, wobei Additive, ausgewählt aus der folgenden Liste: Weichmacher, Säuren, Vernetzungsmittel, Alkalien, Emulgatoren, Antioxidantien, Mittel, die das Verfahren unterstützen, oder eine Mischung davon oder andere Mittel, welche die Bildung der Kapseln oder die Aufnahme der zu verkapselnden Bestandteile erleichtern, in Schritt a) zugegeben werden.

11. Verfahren gemäß einem der Ansprüche 1 bis 10, wobei im Anschluss an Schritt b) ein Homogenisierungsschritt mittels Rühren und/oder Ultraschall durchgeführt wird.

12. Verfahren gemäß einem der Ansprüche 1 bis 11, wobei in Schritt c) das Elektrospinnen oder Elektrosprühen durch Anlegen einer Spannung zwischen 5 und 30 kV durchgeführt wird.

13. Kapseln, die durch das Verfahren nach den Ansprüchen 1 bis 12 erhalten werden können.

14. Verwendung der Kapseln nach Anspruch 13, um chemische Zusammensetzungen oder funktionelle Behältnisse herzustellen.

15. Verwendung der Kapseln nach Anspruch 14, wobei die chemischen Zusammensetzungen aus der Gruppe ausgewählt sind, die aus funktionellen Lebensmittelprodukten, pharmazeutischen Zusammensetzungen, biomedizinischen Zusammensetzungen und nutrazeutischen Zusammensetzungen besteht.

## Revendications

1. Procédé pour obtenir des capsules à partir d'un produit de protéines lactosériques, comprenant les étapes suivantes :
a) dilution du produit de protéines lactosériques dans de l'eau,
b) addition de ce qui suit à la solution de l'étape a) :
i. des ingrédients fonctionnels destinés à être encapsulés qui peuvent être mis en solution ou en suspension dans de l'eau, ou
ii. une solution de l'ingrédient destiné à être encapsulé ;
**caractérisé en ce que** le procédé comprend aussi :
c) l'électro-filage ou l'électro-pulvérisation ou le filage par soufflage ou la pulvérisation par soufflage de la solution produite dans l'étape b), dans lequel
∘ l'électro-filage ou l'électro-pulvérisation est réalisé avec une distance entre le capillaire et le support comprise entre 2 et 50 cm, une plage de vitesse de déposition par aiguille de 0,01 à 10 ml/h et une tension comprise entre 0,1 et 1000 kV, et
∘ le filage par soufflage ou la pulvérisation par soufflage est réalisé avec une distance entre le capillaire et le support comprise entre 2 et 50 cm, une plage de vitesse de déposition par aiguille de 0,01 à 10 ml/h, et par application d'un flux de gaz pressurisé compris entre 200 et 300 m/s.

2. Procédé selon la revendication 1, dans lequel le produit à base de protéines lactosériques est un concentré de protéines lactosériques issues de mammifères.

3. Procédé selon la revendication 2, dans lequel le produit à base de protéines lactosériques est un concentré de protéines lactosériques issues de lait de vache, de brebis ou de chèvre.

4. Procédé selon la revendication 3, dans lequel le produit à base de protéines lactosériques provient d'un concentré de protéines lactosériques issues de lait de vache.

5. Procédé selon l'une quelconque des revendications 1 à 4, dans lequel le pourcentage en poids de la protéine dans la solution est situé dans la plage allant de 0,1 à 99 %.

6. Procédé selon l'une quelconque des revendications 1 à 5, dans lequel le pourcentage en poids de la protéine dans la solution est situé dans la plage allant de 10 à 70 %.

7. Procédé selon l'une quelconque des revendications 1 à 6, dans lequel les ingrédients sont des ingrédients fonctionnels choisis dans le groupe constitué par les antioxydants, les probiotiques, les cellules pour régénération osseuse et tissulaire, les prébiotiques, les symbiotiques, les fibres, l'acide oléique, les acides gras polyinsaturés, les huiles de poisson, les phytostérols, les phyto-œstrogènes, les ingrédients fonctionnels de nature protéique, les nutraceutiques, les enzymes ou protéines ayant une valeur technologique choisies parmi la lactoferrine, l'ovotransferrine, la lactoperoxydase, le lysozyme, la protéine de soja, l'immunoglobuline ou l'une quelconque de leurs combinaisons.

8. Procédé selon l'une quelconque des revendications 1 à 7, dans lequel les ingrédients fonctionnels sont choisis dans le groupe constitué par :
a) le β-carotène,
b) les bifidobactéries et les ferments lactiques,
c) les cellules ayant un intérêt biomédical pour la régénération osseuse et tissulaire,
d) les acides gras polyinsaturés,
e) les enzymes et d'autres protéines ayant une valeur technologique choisies parmi la lactoferrine, l'ovotransferrine, la lactoperoxydase, le lysozyme, la protéine de soja, l'immunoglobuline,
f) les peptides bioactifs choisis parmi les peptides antihypertenseurs et antimicrobiens.

9. Procédé selon l'une quelconque des revendications 1 à 8, dans lequel d'autres biopolymères choisis dans la liste suivante : hydrates de carbones, protéines et lipides, sont ajoutés dans l'étape a).

10. Procédé selon l'une quelconque des revendications 1 à 9, dans lequel des additifs choisis dans la liste suivante : plastifiants, acides, agents de réticulation, alcalis, émulsionnants, antioxydants, agents qui assistent un processus, ou l'un quelconque de leurs mélanges, ou d'autres agents qui facilitent la formation des capsules ou l'incorporation des ingrédients destinés à être encapsulés, sont ajoutés dans l'étape a).

11. Procédé selon l'une quelconque des revendications 1 à 10, dans lequel, après l'étape b), une étape d'homogénéisation est réalisée au moyen d'une agitation et/ou d'ultrasons.

12. Procédé selon l'une quelconque des revendications 1 à 11, dans lequel, dans l'étape c), l'électro-filage ou l'électro-pulvérisation est réalisé par application d'une tension comprise entre 5 et 30 kV.

13. Capsules pouvant être obtenues par le procédé des revendications 1 à 12.

14. Utilisation des capsules de la revendication 13 pour préparer des compositions chimiques ou des récipients fonctionnels.

15. Utilisation de capsules selon la revendication 14, dans laquelle les compositions chimiques sont choisies dans le groupe constitué par les produits alimentaires fonctionnels, les compositions pharmaceutiques, les compositions biomédicales et les compositions nutraceutiques.
